# EUROPEAN PATENT APPLICATION

(11) **EP 3 895 751 A1**
(43) Date of publication of application: **20.10.2021**
(21) Application number: 19897119.4
(22) Date of filing: 05.12.2019
(51) Int. Cl.: A61M 16/20

(54) **BREATHING APPARATUS**

(30) Priority: 14.12.2018 JP 2018234581; 25.03.2019 JP 2019056380
(71) Applicant: Teijin Pharma Limited, Tokyo 100-0013 (JP)
(72) Inventor: IGARASHI Satoshi, Tokyo 100-0013 (JP)
(74) Representative: Hoffmann Eitle
(86) International application number: PCT/JP2019/048595
(87) International publication number: WO 2020/122155

(57) **Abstract**

The present invention is an apparatus that gives patency to the respiratory tract to reduce suffocating feeling by maintaining a pressure higher than atmospheric pressure in the nasopharynx during exhalation; an apparatus that is equipped with an air flow part communicating with a nasal mask covering user's nostrils or with user's nostrils and a casing forming a chamber for temporarily holding the exhaled air of the user; and provides a breathing apparatus that is equipped with a first opening provided in the casing for discharging the exhaled air temporarily held in the casing to the outside and an exhalation control valve that releases exhaled air to the first opening side at the start of exhalation of the user and closes the first opening when the pressure in the chamber on the nostril side exceeds a predetermined value due to exhalation.

## Description

### [Technical field]

The present invention relates to a breathing apparatus for treating a patient with a respiratory disease.

### [Background Art]

There are various types of respiratory diseases such as bronchial asthma, chronic obstructive pulmonary disease, interstitial pneumonia, and obstructive sleep apnea syndrome. Among them, the number of patients with chronic obstructive pulmonary disease or obstructive sleep apnea syndrome, each of which is one of obstructive respiratory diseases, has increased in recent years. Chronic obstructive pulmonary disease is a lifestyle-related disease of the lungs that makes it difficult to breathe due to damage in the air passage for respiration such as bronchi or lungs, and is closely related to smoking. For the diagnosis of chronic obstructive pulmonary disease, a spirometer is generally used to measure the state of ventilatory function, and presence or degree of ventilatory dysfunction is diagnosed by measuring indicators such as vital capacity, forced vital capacity, forced expiratory volume in one second, and forced expiratory volume one second percent.

Obstructive sleep apnea syndrome is caused by narrowing of the upper respiratory tract during sleep, which is presumably caused mainly by obstruction of the respiratory tract by lowering the base of the tongue or soft palate due to fat deposition around the neck and throat, enlarged tonsils, and muscle relaxation. The symptom is that the respiratory airflow in the mouth and nose during sleep causes multiple times of stop or drop to a certain ventilation amount or less for a certain period of time: the symptom of respiration stop for 10 seconds or more is referred to as apnea and that of continuation of reduced ventilation amount to 50% or less of the normal level for 10 seconds or more is referred to as hypopnea. The symptom is diagnosed by the apnea-hypopnea index expressed by the number of times of apnea and hypopnea per hour. It is known that the symptom of this number of 30 or more is evaluated as severe: frequent excessive drowsiness during the day and further an increased risk of developing hypertension, stroke, and cardiovascular diseases such as myocardial infarction. Such obstructive sleep apnea syndrome is usually diagnosed using a simple sleep-respiration monitor, polysomnography, or the like.

Obstructive sleep apnea syndrome is a common disease especially in obese middle-aged and older men, but patients often do not notice the symptoms that occur during sleep at night, and the syndrome has become a social problem as one of the causes of traffic accidents in recent years, which requires countermeasures.

Among various treatment methods proposed for obstructive sleep apnea syndrome, nasal continuous positive airway pressure therapy (CPAP therapy) is widely used. CPAP therapy is to prevent respiratory tract obstruction by sending air to the respiratory tracts continuously, and specifically to keep the respiratory tract patent at night by sending pressurized air from a pressurized air generator (CPAP device) used for CPAP therapy to the respiratory tracts via an air tube and nasal mask, which prevents respiratory tract obstruction during sleep and thus prevents the occurrence of apnea.

Further, PLT 1 discloses a nasal mask provided with a plunger that opens at a predetermined pressure or higher during exhalation, and discloses techniques to reduce the symptoms of obstructive sleep apnea syndrome by giving a high airflow resistance during exhalation, thus keeping a pressure higher than atmospheric pressure to the nasopharynx, and thus giving patency to the respiratory tracts. Further, PLT 2 discloses a breathing apparatus for the nasal cavity capable of adhering a structure based on the technique of a one-way valve to the outer periphery of the nostril.

### [Citation List]

### [Patent Literature]

[PTL 1]
   U.S. Patent No. 5,649,533
[PTL 2]
   Japanese Patent No. 5230202

### [Non-Patent Literature]

[NPL 1]
   Journal of Clinical Sleep Medicine 2011 Vol. 7, pp. 449-453B "Long-Term Use of a Nasal Expiratory Positive Airway Pressure (EPAP) Device as a Treatment for Obstructive Sleep Apnea (OSA)"
[NPL 2]
   Respirology (2017) vol.22(8), p1500-1507 "Treating OSA: Current and emerging therapies beyond CPAP"

### [Summary of Invention]

### [Technical Problem]

Treatment of obstructive sleep apnea syndrome should normally require high respiratory tract pressure to open the respiratory tracts only when the respiratory tracts are obstructed during exhalation that has caused an apnea condition. However, in CPAP therapy, since the CPAP device constantly sends air with a pressure higher than atmospheric pressure to the respiratory tracts, the patient may feel uncomfortable at the start of treatment and may experience symptoms such as sleeplessness. In addition, CPAP therapy may be interrupted due to the troublesomeness of attaching/detaching the nasal mask.

Further, as in the apparatuses described in PTL 1 and PTL 2, a technique of applying respiratory tract pressure during every exhalation period always forces the patients during sleep to exhale against high airflow resistance even in the absence of occurrence of sleep apnea, thus disables the patients from sufficient exhalation. NPL 1 reported that the nasal breathing apparatus using the technique of PTL 2 was used in clinical research and following adverse events were observed: feeling of difficulty in breathing, nasal discomfort, dry mouth, headache, and insomnia. Especially in the case of patients who are not accustomed to treatment using such an apparatus, some patients cannot continue the treatment due to suffocating feeling. For these reasons, it is difficult to improve the therapeutic effect by applying a high pressure equivalent to the positive pressure used in CPAP therapy. Therefore, NPL 2 reports that the nasal respiratory apparatus using the technique described in PTL 2 is effective for patients with a mild to moderate apnea-hypopnea index.

The present invention is to provide a breathing apparatus that assists respiration and reduces suffocating feeling during exhalation for the patients with obstructive respiratory diseases, more specifically obstructive sleep apnea syndrome, while adopting a technique of applying a pressure higher than atmospheric pressure to the upper respiratory tract during exhalation to give patency to the respiratory tract.

### [Solution to Problem]

The present invention provides an apparatus to give patency to the respiratory tract by controlling the timing for discharging user's exhaled air to outside the system to release the exhaled air as it flows in the early stage of exhalation and maintain a high respiratory tract pressure in the latter half of the exhalation by suppressing the release of exhaled air to outside the system. Specifically, the apparatus is equipped with a casing forming a chamber for temporarily holding the user's exhaled air through a ventilation part communicating with a nasal mask covering a nostril part or with the nostril part, a first opening provided in the casing for discharging outside the exhaled air temporarily held in the chamber, and an exhalation control valve for controlling release of exhaled air from the first opening depending on the pressure in the chamber.

The exhalation control valve is characterized in that, while the exhaled air is released to the first opening side at the start of user's exhalation, the valve closes when the pressure in the chamber exceeds a predetermined value, and is equipped with a pressure-deformable elastic body provided with a first hole through which the exhaled air flows and a wall surface part with which the hole edge of the first hole is in contact when the elastic body is pressure-deformed toward the first opening side of the casing.

### [Advantageous Effects of Invention]

According to the present invention, there can be provided a breathing apparatus for treating a patient with a respiratory disease, more specifically a patient with obstructive sleep apnea syndrome, wherein the patient's suffocating feeling is reduced.

### [Brief Description of Drawings]

Fig. 1 shows a schematic view of a state when a user is at inhalation in the configuration of the breathing apparatus according to the first embodiment of the present invention. Fig. 2 shows a schematic view of a state when the user starts exhalation in such an apparatus configuration, and Fig. 3 shows a schematic view of a state when the exhalation is further continued.

Fig. 4 shows a schematic view of a state when a user is at inhalation in the configuration of the breathing apparatus according to the second embodiment of the present invention. Fig. 5 shows a schematic view of a state when the user starts exhalation in such an apparatus configuration, and Fig. 6 shows a schematic view of a state when the exhalation is further continued, and Fig. 7 shows a schematic view of a state when the user starts inhalation. When the user continues to inhale, the state shown in Fig. 4 is given.

Fig. 8 shows a time-series pattern of flow rate/pressure measured using a respiration simulator in the apparatus configuration according to the second embodiment of the present invention.

Figs 9 to 12 show schematic views of a state when the user is at inhalation in the apparatus configuration respectively according to the third to sixth embodiments of the present invention.

Fig. 13 shows a schematic view of a state when a user is continuing to exhale during exhalation in the apparatus configuration according to the seventh embodiment of the present invention. Fig. 14 shows a perspective view of the casing part at the wall surface part side through the cross section of a part of the casing along the A-A' line in Fig. 13.

Figs. 15 and 16 show schematic views of a state when the user is at inhalation in the apparatus configuration according to the eighth and ninth embodiments of the present invention.

Fig. 17 shows a schematic view of the apparatus configuration of the breathing apparatus according to the tenth embodiment of the present invention, and Figs. 18 and 19 show a structure diagram of a support plate provided in the apparatus.

Figs. 20 to 22 show time-series patterns of sound pressure levels of each apparatus.

### [Description of Embodiments]

### [Apparatus according to the first embodiment]

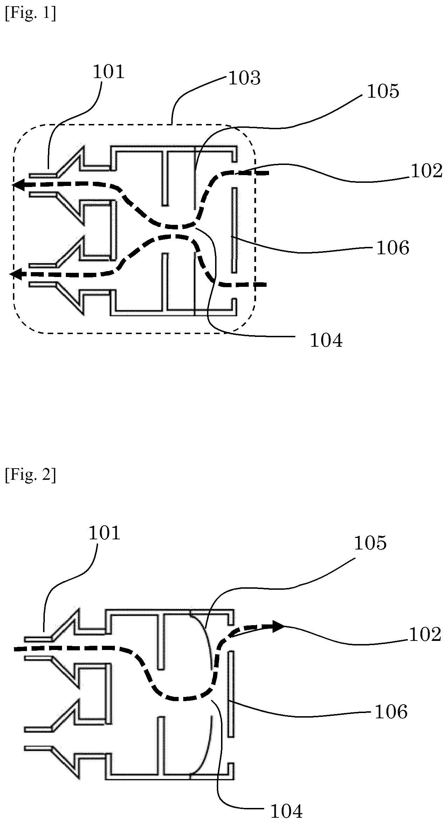

The configuration of the breathing apparatus according to the first embodiment of the present invention is shown in Figs. 1 to 3.

The breathing apparatus is equipped with a air flow part 101 which is inserted into the user's nostril, or is in close contact with or covers the nostril part and allows the user's respiration gas to flow through, a casing 103 forming a chamber temporarily holding the exhaled air inside, and a first opening 102 provided in the casing 103 for discharging exhaled air into the atmosphere, and is equipped inside the casing 103 with an elastic body 105 made of an elastic membrane having a first hole 104 and a wall surface part 106 at a position where the first hole 104 is closed by the deformed elastic body 105 due to expiratory pressure.

The air flow part 101 is inserted into the nostril of the user and is a tubular member in close contact with the inside of the nostril, and introduces inhaled air into the nostril and exhaled air into the chamber of the casing 103 in accordance with the user's respiration. The air flow part 101 may be any member as long as it is sealed from the outside air and communicates with the user's nostrils, and the following can be used such as a prong shown in Fig. 1 in close contact with the nostril that is used in CPAP therapy, a nasal mask that covers a nose including the nostrils, or a tube-shaped member to be inserted into the nostril.

The first opening 102 provided in the casing 103 is an opening for discharging the user's exhaled air charged inside the casing 103 to the outside of the casing 103, the number of openings may be one or more, and the size and arrangement of the openings can be designed as appropriate. In order to prevent foreign matter outside from entering inside, the first opening 102 may have a large number of micropores or a net-like filter may be provided on the first opening 102.

An elastic body 105 is provided inside the casing 103 between the air flow part 101 and the first opening 102. The elastic body 105 is characterized to be a member, which is deformed by the pressure of the exhaled air of the user charged into the chamber of the casing 103, of such as a bellows structure, a piston structure, and an elastic membrane, and provided with at least one first hole 104 at a portion deformed by the pressure. Since the elastic body 105 needs to be deformed by the pressure of the user's exhaled air, the first hole 104 needs to have a total opening area sufficient to give airflow resistance to the expiratory pressure. Therefore, the total opening area is preferably 1000 mm² or less, more preferably 500 mm² or less, and further preferably 300 mm² or less. The number of the first holes 104 may be one or more in the central portion of the elastic body 105.

The elastic body 105 is preferably an elastic membrane because it has a simple structure and thus can be easily miniaturized. As the material of the elastic membrane, rubber is suitable, and silicone rubber is preferable. The thickness of the elastic membrane is, from the viewpoint of easy deformation by exhalation and durability of the elastic membrane, though depending on the elastic modulus of the material of the elastic membrane, preferably 1000 µm or less and 10 µm or more, and more preferably 500 µm or less and 20 µm or more. In the case of a cylindrical casing, a ring-shaped silicone elastic membrane having a first hole 104 at the center can be used as the elastic body 105.

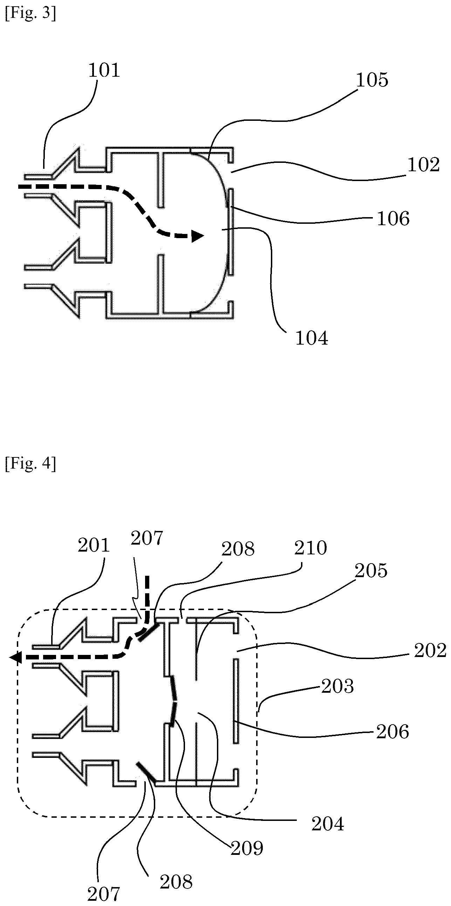

In the present embodiment, the wall surface part 106, which is an inner wall surface having the first opening 102 of the casing 103, is configured to face the first hole 104. As shown in Fig. 3, the wall surface part 106 is installed so that the hole edge of the first hole 104 comes into contact with the wall surface part 106 when the elastic body 105 is deformed by the expiratory pressure during exhalation of the user. The shape of the inner wall surface of the wall surface part 106 is not limited to a flat surface, and may be a concave curved surface or a convex curved surface, and may only be a planar shape that enables the hole edge of the first hole 104 to come into contact with the wall surface part 106.

Immediately after the start of exhalation of the user, the exhaled air is released outside from the air flow part 101 through the first hole 104 of the elastic body and the first opening 102 of the casing 103, and when the expiratory pressure causes the hole edge of the first hole 104 of the elastic body 105 to come into contact with the wall surface 106, the hole closes and the release of the exhaled air is suppressed, resulting in an increase in the respiratory tract pressure during exhalation. The pressure of the chamber of the nostril part side in the casing, in which the hole edge of the first hole of the elastic body is in contact with the wall surface part and the first opening is closed, can be set freely by changing the distance between the elastic body and the wall surface part. The pressure is set in the range of 4 to 20 cmH₂O, which is the respiratory condition used in CPAP therapy, and can be set, depending on the respiratory condition of the user, to a value such as 4 cmH₂O or more, and 10 cmH₂O or more to realize the effect of reducing suffocating feeling caused by release of exhaled air at the start of exhalation.

When arrangement of the elastic body 105 and the first opening 102 is such that when the hole edge of the first hole 104 comes into contact with the wall surface part 106 and the first hole 104 of the elastic body 105 and the first opening 102 overlap each other, the effect of increase in airflow resistance by exhaled air cannot be attained. Therefore, the first opening 102 is preferably located on the outer peripheral part of the hole edge of the first hole 104 to avoid the overlapping of the two. However, in order to adjust the airflow resistance, a part of the first opening 102 and a part of the first hole 104 may overlap each other to such an extent that does not reduce the effect of increase in airflow resistance.

In the breathing apparatus according to the present embodiment, the elastic body 105 starts its deformation by the start of exhalation of the user as shown in Fig. 2. An expiratory airflow is generated during the state from the start of exhalation until the state in which the hole edge of the first hole 104 and the wall surface part 106 come into contact with each other due to sufficient deformation of the elastic body 105. As the expiratory time passes, the hole edge of the first hole 104 and the wall surface part 106 approach each other, resulting in a decrease in the flow path area and the expiratory airflow rate, and at the same time, in an increase in pressure in the chamber of the casing 103, which is the expiratory pressure. In the latter half of exhalation after the further continued exhalation state, as shown in Fig. 3, the hole edge of the first hole 104 comes into contact with the wall surface part 106 and the exhaled air is shut out, resulting in the increase in airflow resistance of the exhaled air and thus in a large increase in the pressure in the chamber of the casing 103 which is equivalent to the internal pressure applied to the nasopharynx of the user. Therefore, it is possible to keep the airflow resistance of the exhaled air low at the start of exhalation, and to apply a pressure higher than atmospheric pressure to the nasopharynx as exhalation continues. As a result, the respiratory tract of the user can be patent to reduce the symptoms of obstructive sleep apnea syndrome, and the pressure at the initial stage of exhalation can be decreased to reduce the suffocating feeling of the user.

On the other hand, when the exhalation of the user is completed and the pressurization of the chamber of the casing 103 is stopped, the elastic body 105 returns to its original shape. During inhalation, the hole edge of the first hole 104 and the wall surface part 106 are in a non-contact state, and the exhaled air in the chamber of the casing 103 which is pressurized and filled through the first hole 104 and the first opening 102 is released and the air outside the casing 103 is introduced. During inhalation, the inspiratory air is introduced from the first opening 102 to the nostril through the first hole 104 and the air flow part 101. In the breathing apparatus according to the present embodiment, there is shown an integrated configuration in which an exhalation control valve, which is provided with an elastic membrane 105 having the first hole 104 and a wall surface part 106 to be in contact with the hole edge of the first hole, is incorporated into the casing 103. However, the exhalation control valve may be configured as an independent member to be incorporated into the casing.

### [Apparatus according to the second embodiment]

The configuration of the breathing apparatus according to the second embodiment of the present invention is shown in Figs. 4 to 7. Such a breathing apparatus is equipped with those similar to the first embodiment such as an air flow part 201 inserted into and in close contact with the user's nostril and an elastic body 205 made of an elastic membrane having a first hole 204 in a chamber of a casing 203 which has a first opening 202 to release exhaled air into the atmosphere, and in addition, an intake hole 207, an intake valve 208 (one-way valve), an exhaust valve 209 (one-way valve), and a second opening 210 to release a part of exhaled air into the atmosphere.

In the present embodiment, the casing 203 is equipped with an intake hole 207 provided between the air flow part 201 and the elastic body 205, and an intake valve 208 which is a one-way valve provided in the intake hole 207 to permit air inflow into the chamber of the casing 203. The number of intake holes 207 may be one or more, and preferably, by providing two intake holes 207 corresponding to the two nostrils as shown in Fig. 4, the atmosphere can be inhaled with a small inhalation resistance. The total opening area of the intake hole 207 is desirable to make airflow resistance as small as possible during inhalation, and is preferably 50 mm² or more. On the other hand, during exhalation, since the intake valve 208, which is a one-way valve, is closed, the pressure of the chamber of the casing 203 increases.

Further, as described above, in the first embodiment, the chamber of the casing 103 between the air flow part 101 and the elastic body 105 is in a state in which the exhaled air is pressurized and filled during exhalation, and when the exhalation is completed, the elastic body 105 recovers the original shape and loses contact with the wall surface part 106, and the exhaled air pressurized and filled is released through the gap between the first hole 104 and the first opening 102. However, in the early stage of inhalation, the exhaled air pressurized and filled may flow backward and the exhaled air may be re-inhaled. In the present embodiment, in order to suppress the re-respiration of exhaled air, an exhaust valve 209, which is a one-way valve, is provided in the chamber of the casing 203 between the air flow part 201 and the elastic body 205. The exhaust valve 209 allows the air flow from the air flow part 201 side to the elastic body 205 side, and blocks the air flow in the opposite direction. Therefore, during exhalation, exhaled air can be introduced to the elastic body 205 side to increase the pressure in the chamber of the casing 203, and during inhalation, the high-pressure exhaled air filled in the elastic body side can be suppressed to flow back to the user through the air flow unit 201.

When the exhaust valve 209 is provided, the exhaust valve 209 is closed during inhalation and thus inhalation is not performed through the first hole 204 of the elastic body 205 and the first opening 202 of the casing 203. Therefore, in the breathing apparatus provided with the exhaust valve 209, the intake hole 207 needs to be provided between the air flow part 201 and the elastic body 205 and on the air flow part 201 side with respect to the exhaust valve 209.

Further, in the present embodiment, a second opening 210 is provided between the elastic body 205 of the casing 203 and the exhaust valve 209. The second opening 210 can be provided to allow a part of the exhaled air to escape to the outside of the casing 203 and thus to adjust the airflow resistance during exhalation, that is, to adjust the pressure in the nasopharynx during exhalation, and it may be one or more. In addition, in preparation for excessive expiratory pressure due to exhalation anomalies of the user, the part of the second opening 210 may be made of an elastic material for the opening part to be deformed by pressure or adopt a structure that increases the opening area by attaching a slit structure or a pleated deformation part, and then to facilitate the release of exhaled air. The total opening area of the second opening 210 is required to be smaller than the total opening area of the first hole 204 so as not to affect the deformation of the elastic body 205 due to exhalation and the contact between the first hole 204 and the wall surface part 206. It is preferably 10 mm² or less and more preferably 5 mm² or less.

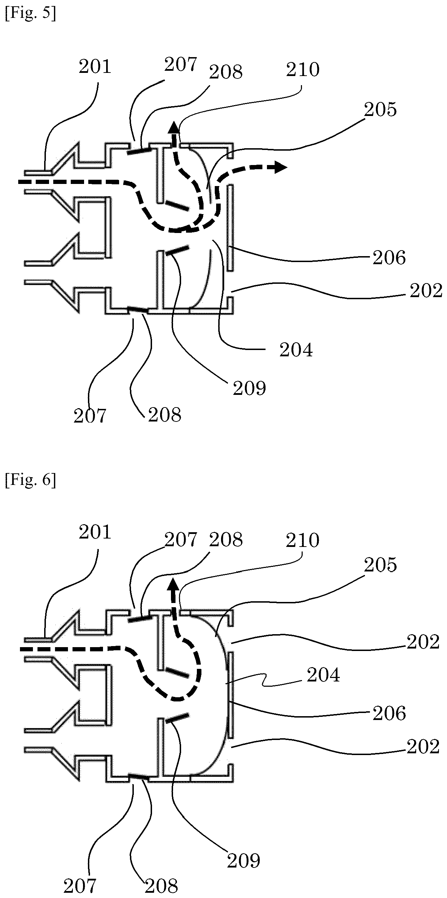

Fig. 5 shows the initial stage of exhalation in the breathing apparatus according to the present embodiment. In this case, the intake valve 208 is closed by the exhaled air, and the exhaled air is released not therefrom, but from the first opening 202 and the second opening 210 by the exhaust valve 209 opened by the expiratory pressure.

Fig. 6 shows the latter half stage of exhalation. When the elastic body 205 comes into contact with the wall surface part 206 of the casing due to expansion deformation, since the hole edge of the first hole 204 formed in the elastic body 205 comes into close contact with the wall surface part 206 to cause the closure of the first hole 204, the exhaled air is released only from the second opening 210. As a result, the expiratory pressure, that is, the pressure in the chamber of the casing increases. The increased pressure in the chamber can be adjusted by the size of the second opening 210, and a high internal pressure can be given by making the opening with a small opening area.

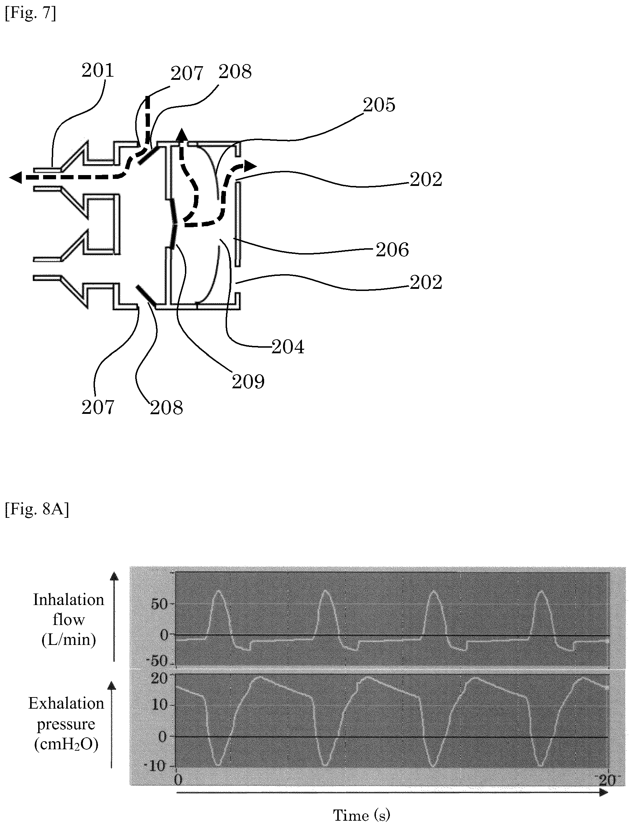

Subsequently, once the inhalation starts, the intake valve 208 is opened as shown in Fig. 7, which enables easy inhalation, and at the same time, the exhaled air remaining in the chamber on the elastic body 205 side in the casing is quickly releasereleased into the atmosphere, as the elastic body 205 shrinks and the first hole 204 and the first opening 202 communicate.

### [Experimental Example 1]

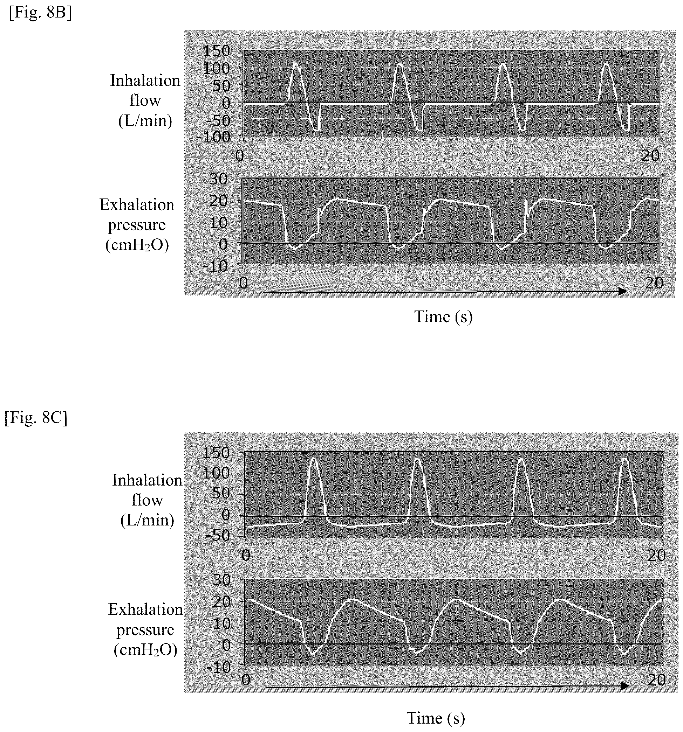

In the breathing apparatus according to the second embodiment, the air flow part 201 was connected to the ASL5000 respiration simulator manufactured by INGMAR MEDICAL via a flow path imitating the inside of the nasopharynx of an adult, and artificially generated respiration was used to measure the flow rate/pressure pattern, which is shown in Fig. 8 The respiration simulator was set with the parameters such as breath rate: 12 bpm, expiratory muscule pressure: 17 cmH₂O, inspiratory muscule pressure: 20 cmH₂O, inspiratory rise time: 10% of one respiratory cycle, expiratory rise time: 25%, inspiratory release time: 15%, expiratory release time: 5%, and expiratory hold: 45%.

### [Example 1]

Silicone rubber with a thickness of 50 µm and has an elastically deformable part with a diameter of 50 mm is used as the membrane of the elastic body 205 of the breathing apparatus, the diameter of the first hole 204 is set to 16 mm, and the distance between the elastic body 205 and the wall surface part 206 is set to 6 mm, and the casing 203 is provided with 6 holes each with a diameter of 6 mm, as the first opening 202, around the first hole 204, when viewed perpendicularly to the surface of the elastic body 205, under which configuration the experiment was performed. The total opening area of the intake hole 207 was 280 mm². Further, the intake valve 208 was a membrane type on-off valve using a silicone rubber membrane with a thickness of 100 µm. The opening area of the second opening 210 was 0.8 mm². The results of the measurement of the flow rate/pressure pattern in this example are shown in Fig. 8A.

### [Example 2]

In the breathing apparatus of Example 1, a polyethylene terephthalate film with a thickness of 50 µm and having a hole with a diameter of 16 mm at the same position of the first hole 204 was provided for supporting the elastic membrane on the respiration simulator side of the elastic membrane. Further, the distance between the elastic body 205 and the wall surface part 206 was set to 7.3 mm. The results of the measurement of the flow rate/pressure pattern in this example are shown in Fig. 8B.

### [Comparative Example 1]

As a Comparative Example, breathing apparatus of Example 2 is set up, as in the conventional technique, to have a structure in which the airflow resistance during exhalation is larger than the airflow resistance during inhalation: the first opening 202 was closed and the second opening 210 was set to have the opening area of 6.5 mm². The results of the measurement of the flow rate/pressure pattern in this comparative example are shown in Fig. 8C.

These results show the following. In Example 1, an expiratory airflow was generated at the initial stage of exhalation, but the expiratory airflow decreased in the middle of the exhalation, and at the same time, the expiratory pressure, that is, the pressure in the chamber of the casing 203 increased. That is, it can be confirmed that discharging air to the outside of the system at the initial stage of exhalation allows to suppress an increase in the expiratory pressure from the initial stage of exhalation. Further, in Example 2, it can be confirmed that, as compared with Comparative Example 1, a higher expiratory airflow is observed in the early stage of exhalation, and after that, the maximum expiratory pressure becomes equivalent, but a higher pressure is maintained at the end of exhalation. This shows that it is possible in the early stage of exhalation to reduce the pain by discharging the exhaled air out of the system, from the middle stage of the exhalation to increase the expiratory pressure, and at the final stage of exhalation, which is effective for respiratory tract patency, to increase the pressure in the respiratory tract. This exhalation pattern, though varies depending on the exhalation intensity, can be designed to fit various patterns by adjusting the distance between the elastic body 205 and the wall surface part 206, the size of the first hole 204, and the size of the second opening 210.

### [Apparatus according to the third embodiment]

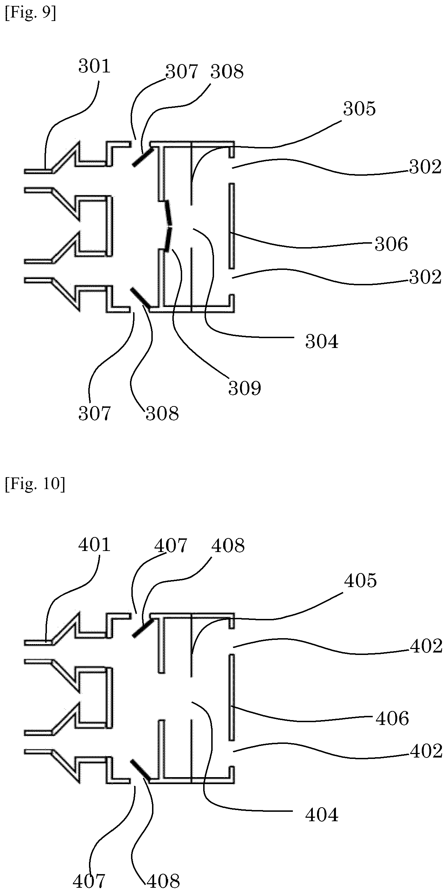

The breathing apparatus shown in Fig. 9 according to the third embodiment has a configuration in which the second opening 210 is omitted from the breathing apparatus according to the second embodiment. As for the second opening 210 of the embodiment of Fig. 4, the smaller the opening area, the higher the pressure at the end of exhalation, and at the extremity of it, the second opening 210 can be eliminated. In the third embodiment, after the final stage of exhalation, the first hole 304 and the first opening 302 communicate as the elastic body 305 shrinks, and with the start of inhalation, the exhaled air remaining in the chamber on the elastic body 305 side of the casing is also rapidly released to the atmosphere.

### [Apparatus according to the fourth embodiment]

The breathing apparatus shown in Fig. 10 according to the fourth embodiment has a configuration in which the exhaust valve 309 is further omitted from the breathing apparatus according to the third embodiment. In this case, though when the intake valve 408 is opened during inhalation, a part of the exhaled air remaining in the chamber of the casing is rebreathed together with the air flowing through the intake hole 407 from the outside, the structure of the apparatus is simplified.

### [Apparatus according to the fifth embodiment]

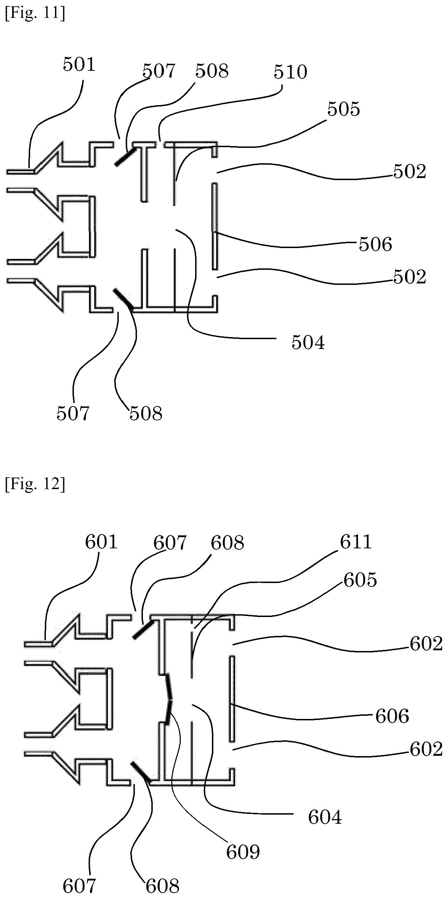

The breathing apparatus shown in Fig. 11 according to the fifth embodiment has a configuration in which the exhaust valve 209 is omitted from the breathing apparatus according to the second embodiment. In this case also, though when the intake valve 508 is opened during inhalation, a part of the exhaled air remaining in the chamber of the casing is rebreathed together with the air flowing through the intake hole 507 from the outside, the structure of the apparatus is simplified. Further, the pressure in the chamber of the casing, which is the expiratory pressure, can be adjusted by the second opening 510.

### [Apparatus according to the sixth embodiment]

The breathing apparatus shown in Fig. 12 according to the sixth embodiment has a configuration in which the second opening 210 is not provided in the breathing apparatus according to the second embodiment and instead the second hole 611, which has the same function as the second opening 210, is provided in the elastic body 605. When the elastic body 605 is made of an elastic membrane, the amount of deformation thereof increases toward the center of the elastic membrane, that is, the center of the cross section in the air flow direction of the casing. Therefore, by providing the second hole 611 at the periphery part of the elastic body 605 and in the vicinity where the elastic body 605 is fixed to the casing, even when the elastic body 605 expands during exhalation for the hole edge of the first hole 604 to come into contact with the wall surface part 606, the exhaled air can be still released from the first opening 602 through the second hole 611. According to this embodiment, since the second hole 611 is provided in the casing, the user does not directly touch the second hole 611, and thus harmful effects such as blockage due to dust or the like are reduced compared with the case where the opening is provided on the outer surface of the casing. It is also possible to have a configuration in which the second opening 210 in the second embodiment and the second hole 611 are both provided.

### [Apparatus according to the seventh embodiment]

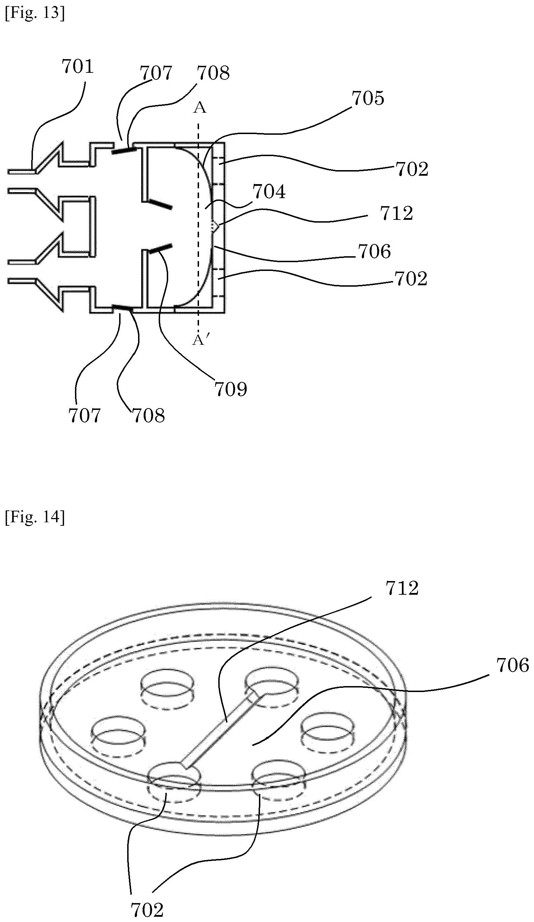

The breathing apparatus shown in Fig. 13 according to the seventh embodiment has a configuration in which the second opening 210 is not provided and a groove 712 connected to the first opening 702 is provided on the wall surface part 706 in the breathing apparatus according to the second embodiment, and shows a schematic diagram when the elastic body 705 is deformed as the user continues exhalation. Further, Fig. 14 is a perspective view of a part of the casing on the wall surface part 706 side in the cross section taken along the line indicated by A-A' in Fig. 13. The space, which is formed by the groove 712 connected to the first opening 702 provided on the wall surface part 706 and the elastic body 705 when the elastic body 705 is deformed and the hole edge of the first hole 704 comes into contact with the wall surface part 706, becomes a communication passage for discharging exhaled air and can provide the same effect as that of the second opening 210. Furthermore, since the elastic body 705 is deformed to enter the groove 712 and thus the opening area of this communication passage changes depending on the amount of deformation of the elastic body 705, a pressure/opening area relationship is obtained which is different from an opening area simply formed into a constant size. Therefore, the exhalation intensity changes the cross-sectional area of the space, by which way the expiratory pressure can be controlled.

Fig. 14 shows that when the elastic body 705 expands and deforms and comes into contact with the wall surface part 706, the elastic body 705 and the groove 712 form a communication passage having a triangular cross section. In the present embodiment, the cross section of the groove 712 is shown as a triangle, but the cross section is not limited to this, and various shapes can be selected such as a quadrangle, a polygon, a trapezoid, and a semicircle.

Further, in the present embodiment, an example is illustrated in which six first openings 702 are provided and the grooves 712 are provided over two of them, but the present invention is not limited to this, and one first opening 702 or two or more holes may be provided, and the groove 712 may be formed so that the first hole 704 communicates with one of a plurality of first openings 702. Alternatively, the groove 712 may be formed from a plurality. Further, the cross-sectional shape of the groove 712 does not have to be a constant shape along the longitudinal direction. For example, by reducing the cross-sectional area as it approaches the first opening 702, the closer the body 705 comes to the wall surface part 706 due to expansion deformation of the elastic body 705, the smaller the cross-sectional area of the communication passage formed by the groove 712 and the elastic body 705 becomes, and then the expiratory pressure can be increased. It is also possible to have a groove 712 and a second opening 210 in the second embodiment and/or a second hole 611 in the sixth embodiment at the same time.

### [Apparatus according to the eighth embodiment]

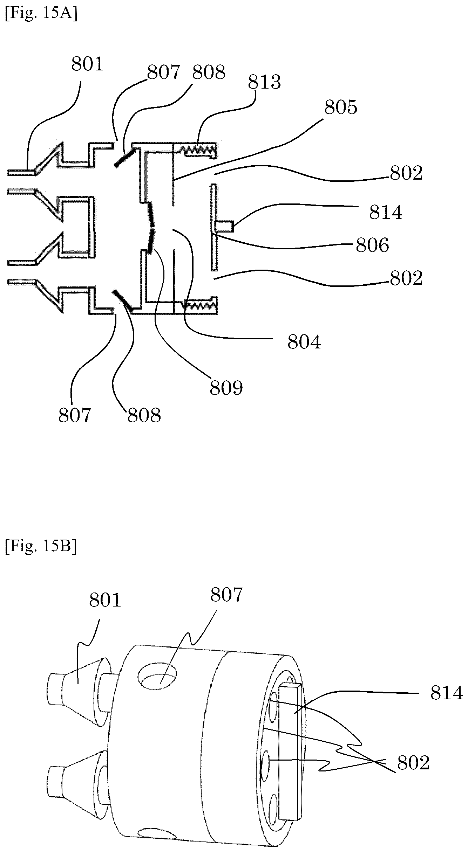

The breathing apparatus shown in Figs. 15A and 15B according to the eighth embodiment shows an embodiment in which a distance adjustment mechanism is provided to adjust the distance between the elastic body 805 and the wall surface part 806 by connecting the casing and the wall surface part 806 with the rotary screw 813 in the breathing apparatus according to the third embodiment. In the present embodiment, the casing has an opening on the wall surface part 806 side, and a female screw is provided on the inner wall of the opening. The wall surface part 806 is provided with a ring-shaped convex part protruding from the outer edge portion, and a male screw is provided on the outer peripheral surface of the ring-shaped convex part. The female screw of the casing and the male screw of the wall surface part 806 are fastened to construct the distance adjustment mechanism using the rotary screw 813. The user can adjust finely the distance between the wall surface part 806 and the elastic body 805 by pinching the adjustment knob 814 provided on the wall surface part 806 and rotating the ring-shaped convex part with respect to the casing. Thereby, the position of the wall surface part 806 can be adjusted depending on the respiratory intensity of the user. When the expiratory pressure of the user is low and an increase is thus required in the airflow resistance of the exhaled air, the distance between the wall surface part 806 and the elastic body 805 can be reduced to exhibit the effect of increase in airflow resistance of the exhaled air.

Though the present embodiment adopted a screw-shaped structure for the distance adjusting mechanism, the present invention is not limited to this. For example, the following structure may be adopted: a structure in which an O-ring or the like is provided instead of a screw between the inner wall of the opening of the casing and the ring-shaped convex part of the wall surface part 806, the inner wall and the ring-shaped convex part are supported via the O-ring or the like provided therebetween, and the position of the wall surface part 806 can be adjusted by pulling out or pushing in the ring-shaped convex part, or a structure that enables the position of the elastic body 505 inside the casing to vary. Further, the distance adjusting mechanism may adopt a structure in which a plurality of replacement parts having different distances between the elastic body 805 and the wall surface part 806 is prepared and the part is replaced to have a desired distance between the elastic body 805 and the wall surface part 806. However, in this case, the mechanism must be disassembled and then assembled for the adjustment of the distance between the elastic body 805 and the wall surface part 806, and in addition, for the fine adjustment of the distance, there must be prepared various replacement parts having small differences in the distance between the elastic body 805 and the wall surface part 806.

### [Apparatus according to the ninth embodiment]

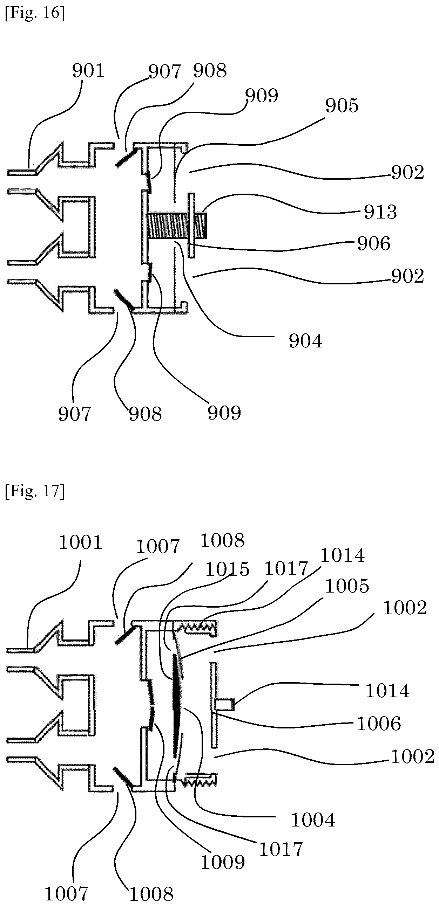

The breathing apparatus shown in Fig. 16 according to the ninth embodiment has a configuration in which the breathing apparatus according to the third embodiment is changed to provide the exhaust valve 909 at a position other than the central axis of the casing and to provide a distance adjusting mechanism using a rotary screw 913 which is connected to the wall surface part 906 at the center and enables the adjustment of the distance between the elastic body 905 and the wall surface part 906. In the present embodiment, a male screw is provided on a shaft extending from the center of the casing toward the wall surface part 906. Further, a female screw for fastening thereto is provided on the inner surface at the center of the wall surface part 906. The user can finely adjust the distance from the elastic body 905 by rotating the wall surface part 906. Accordingly, the position of the wall surface part 906 can be adjusted depending on the respiratory intensity of the user. That is, when an increase is required in the airflow resistance of the exhaled air even if the expiratory pressure of the user is low, the distance between the wall surface part 906 and the elastic body 905 can be reduced to exhibit the effect of increase in the airflow resistance of the exhaled air.

Though the present embodiment also adopted a screw-shaped structure for the distance adjusting mechanism, the present invention is not limited to this. There may be adopted a structure in which an O-ring or the like is provided between a cylinder extending from the center of the casing and the wall surface part 906 having an opening at the center, the cylinder and the wall surface are supported via the O-ring or the like provided therebetween, and the position of the wall surface part 906 can be adjusted by pulling out or pushing in the wall surface part 906.

### [Apparatus according to the tenth embodiment]

In the configuration provided with the elastic membrane that is deformed by the pressure of exhaled air of the patient, a jarring airflow sound may be generated due to deformation of the elastic body and inflow and outflow of the respiratory airflow. In order to reduce this noise, in the breathing apparatus shown in Fig. 17 according to the tenth embodiment, a support plate 1015 is provided as a support member for the elastic body 1005 to come into contact with the surface of the elastic body 1005 opposite to the wall surface part 1006 side.

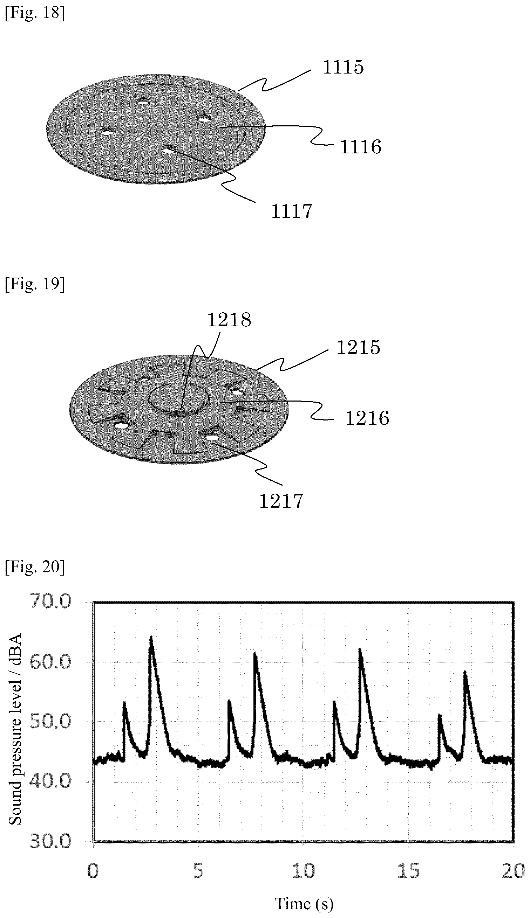

The support member may be a flat plate, and as shown in Figs. 18 and 19, support plates 1115 and 1215 having hemispherical convex surfaces 1116 and 1216 on the elastic body 1005 side can be used. This gives the effect of reducing the slack of the elastic body 1005. For the purpose of reducing slack, the shape is not limited to a hemispherical shape, and may be conical, cylindrical or the like. The material of the support member may be any material as long as having a sufficiently high elasticity as compared with the elastic body 1005, and examples thereof include metal, plastic, and hard rubber.

The support plates 1015, 1115, 1215 are provided with third holes 1017, 1117, 1217 so that the air associated with exhalation can pass through. The elastic body 1005 is deformed by the exhaled air passing through the third hole, and a gap is formed between the elastic body and the support plate, so that the elastic body 1005 expands and deforms and comes into contact with the wall surface part 1006. The third hole is preferably provided with a plurality of holes to avoid as much as possible an uneven expiratory airflow to the elastic body, and a large number of micropores or a mesh-structured member may be used.

The support plate 1115 shown in Fig. 18 was provided with a spherical convex surface 1116 having a radius of curvature of 116 mm and a height of 1.5 mm, on the elastic body 1005 side, and the third hole 1117 was provided as four holes each having a diameter of 4 mm and arranged radially in the outer periphery of the first hole 1004.

The support plate 1215 shown in Fig. 19 was provided with a spherical convex surface 1216 having a radius of curvature of 193 mm and a height of 2.5 mm, and the shape was changed for each third hole 1217 to be lower than the convex surface 1216. Further, a convex shape part 1218 having a diameter smaller than the diameter of the first hole 1004 and a height of 1 mm was provided for the first hole 1004 of the elastic body 1005 to be easily arranged at the center of the spherical surface on the support plate 1215.

### [Experimental Example 2]

Fig. 20 shows the time series pattern of the sound pressure level for the second embodiment measured under the same conditions as in Experimental Example 1. The sound pressure level was measured using a precision sound level meter NL-31 manufactured by Rion Co., Ltd. with an average distance of 10 cm from the tip of the microphone to the part of the first opening 202. The sound pressure level at this time was 64 dBA at the maximum and a high level was shown in the noise range, which was very loud for a breathing apparatus used near the face. For the measurement environment at this time, when the elastic body 205 was removed from the breathing apparatus and the respiration simulator was operated, the sound pressure level of an average of 43 dBA was measured.

From the comparison of the inspiratory flow rate/expiratory pressure pattern in Fig. 8 and the sound pressure level in Fig. 20, the cause of this sound is mainly the airflow sound before the elastic body 205 comes in contact with the wall surface 206 and the airflow sound generated after the final stage of exhalation when the elastic body 205 is separated from the wall surface part 206 and the exhaled air pressurized in the chamber of the casing 203 starts to be released from the first opening 202.

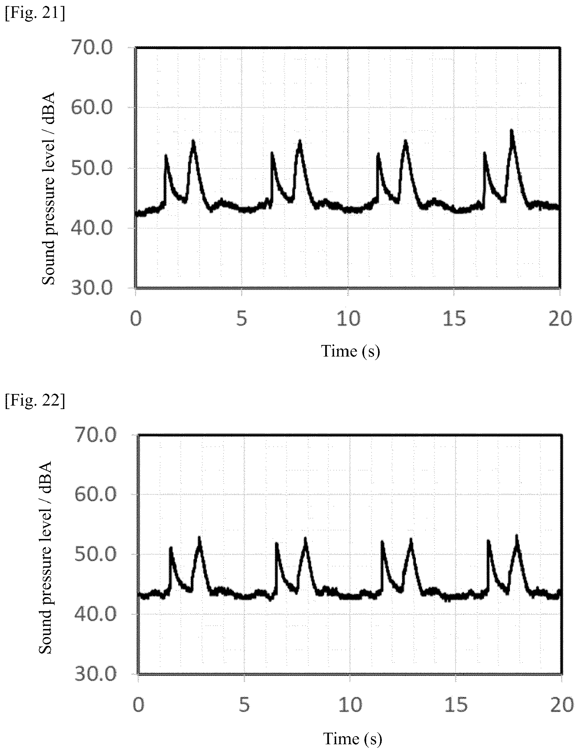

Fig. 21 shows a time-series pattern when the sound pressure level in the breathing apparatus shown in Fig. 17 provided with the support plate 1115 of Fig. 18 was measured under the same measurement conditions. The sound pressure level when the support plate 1115 of Fig. 18 was used is 58 dBA at the maximum, which shows that the sound pressure level is reduced by providing the support plate 1115. Further, it was found that the sound pressure level of the breathing apparatus of Fig. 17 when the support plate 1215 of Fig. 19 was used was 54 dBA at the maximum, which shows that the sound pressure level is further reduced. This is because the periphery of the third hole 1217 in Fig. 19 was lowered from the spherical convex surface 1216 of the support plate 1215 and thus a space was provided to control the flow of exhaled air.

As described above, in the present embodiment, there can be provided a breathing apparatus for treating patients with obstructive respiratory disease, more preferably obstructive sleep apnea syndrome and the apparatus reduces suffocating feeling during exhalation using the technique of applying a pressure higher than atmospheric pressure to the nasopharynx to give the respiratory tract patency.

### [Industrial applicability]

The present invention can contribute to the improvement of the quality of life of patients through utilization of the breathing apparatus for treating patients with obstructive sleep apnea syndrome.

### [Reference Signs List]

101, 201, 301, 401, 501, 601, 701, 801, 901, 1001: Air flow part
102, 202, 302, 402, 502, 602, 702, 802, 902, 1002: First opening
103, 203: Casing
104, 204, 304, 404, 504, 604, 704, 804, 904, 1004: First hole
105, 205, 305, 405, 505, 605, 705, 805, 905, 1005: Elastic body
106, 206, 306, 406, 506, 606, 706, 806, 906, 1006: Wall surface part
207,307,407,507,607,707,807,907,1007: Intake hole
208, 308, 408, 508, 608, 708, 808, 908, 1008: Intake valve
209, 309, 609, 709, 809, 909, 1009: Exhaust valve
210, 510: Second opening
611: Second hole
712: Groove
813, 913: Rotating screw
814, 1014: Adjustment knob
1015, 1115, 1215: Support plate
1116, 1216: Convex surface
1017, 1117, 1217: Third hole
1218: Convex shape part

## Claims

1. A breathing apparatus provided with an air flow part communicating with a nasal mask covering a nostril part of a user or with a nostril part of a user and a casing forming a chamber for temporarily holding exhaled air of the user, comprising:
a first opening provided in the casing to release outside the exhaled air temporarily held in the casing, and
an exhalation control valve that releases exhaled air to the first opening side at the start of exhalation of the user and closes the first opening when a pressure of a chamber on the nostril side due to exhalation becomes higher than a predetermined value.

2. The breathing apparatus according to claim 1, wherein the exhalation control valve is equipped with a pressure-deformable elastic body provided with a first hole through which exhaled air flows and a wall surface part with which the hole edge of the first hole is in contact to close the first hole when the elastic body is pressure-deformed toward the first opening side.

3. The breathing apparatus according to claim 2, wherein the elastic body is an elastic membrane.

4. The breathing apparatus according to claim 3, further comprising a support member for the elastic membrane on the nostril side of the elastic membrane, the support member having a plurality of holes enabling airflow.

5. The breathing apparatus according to claim 2, wherein the wall surface part is composed of a part of an inner wall surface of the casing.

6. The breathing apparatus according to claim 2, further comprising a distance adjusting unit for adjusting the distance between the elastic body and the wall surface part.

7. The breathing apparatus according to any one of claims 1 to 6, wherein the casing or the exhalation control valve has a second opening that does not close regardless of the pressure in the chamber.

8. The breathing apparatus according to any one of claims 1 to 7, wherein the casing is provided with an intake valve that allows inflow of inhaled air from the outside, in the chamber on the nostril side with respect to the elastic body.

9. The breathing apparatus according to any one of claims 1 to 8, wherein the casing is further provided with an exhaust valve to allow exhaled air to flow through toward the elastic body, in the chamber on the nostril side with respect to the elastic body.
